Europäisches Patentamt

European Patent Office

Office européen des brevets

(11) Publication number: **0 470 507 A1**

# EUROPEAN PATENT APPLICATION

(21) Application number: **91112941.9**

(22) Date of filing: **01.08.91**

(51) Int. Cl.5: **A61B 5/00, A61B 10/00**

(30) Priority: **06.08.90 IT 8498190**

(43) Date of publication of application:
**12.02.92 Bulletin 92/07**

(84) Designated Contracting States:
**AT BE CH DE DK ES FR GB GR IT LI LU NL SE**

(71) Applicant: **U.P.F. di Zanieri Ugo Pietro**
**Via Calatafimini 26**
**I-50137 Firenze(IT)**

(72) Inventor: **Lo Sapio, Giovanna**
**Via Palazzina 70**
**I-37138 Verona(IT)**
Inventor: **Zanieri, Ugo Pietro**
**Via Clatafimi 26**
**I-50137 Firenze(IT)**

(74) Representative: **Petraz, Gilberto Luigi**
**GLP S.r.l. Piazzale Cavedalis 6/2**
**I-33100 Udine(IT)**

(54) **Device for the measurement of body temperature.**

(57) The device assisting in a natural method of birth control is based on the measurement of the basal temperature in particular of the variation of the basal temperature caused by the ovulation. The device performs the measurement of the temperature of given zones in an automatic way, without surface contact, in real time and permits examination of women even when wearing light clothing.

The present invention relates to a device for measuring the temperature of the body in a remote way.

More particularly, the present invention relates to a device that performs the measurement of the temperature of the body of a woman in the basal zone in order to check the period of ovulation in the menstrual cycle.

This measurement is carried out in an automatic way, in real time, without touching the body and permits women the choice of wearing light clothes.

This device has its main application in medical and paramedical fields.

It is known that an accurate measurement of body temperature on extended surfaces is an important aid to medicine for checking any anomalies in the body functioning of a person.

As important examples we would mention breast cancer, headaches, neuropathies etc..

This measurement is at present carried out by means of remote thermography, with devices that do not include reference units for temperature in order to obtain a high accuracy.

Furthermore, it is known that control of the period of fertility of a woman during the menstrual cycle is a very important event for family planning.

In fact, nowadays, the problem of the responsibility of procreation is very important for ethical and religious aspects as well as for social and economic ones.

Presently the widely used methods of birth control have considerable negative side effects for the health of women, not excluding a high percentage increase of cancer in women who use oral contraceptives for a large period.

These methods may be briefly summarized:

- oral contraceptive;
- intrauterin devices;
- methods of periodic abstinence;
- condom;
- diaphragm.

All these mentioned methods have considerable disadvantages and drawbacks since:

a) oral contraceptives (pills), based on progesterone and oestrogen, have side effects of varying importance such as irregular bleedings, quite significant weight increase, nausea, headache, nervousness, depression, loss of libido. More important side effects may be felt after use of the pill for a medium period in particular by women with risk factors: these effects may cause a greater chance of death due to cardiocirculatory diseases, including cerebral haemmorhage and miocardio infarct as well as cancer;

b) intrauterin devices have the disadvantages of having to be inserted by a doctor, of monthly checks after menstruation, of a high probability of expulsion, of abdominal pains between periods and of the risk of perforation of the uterus.

c) the methods of periodic abstinence, more easily acceptable for religious reasons, are the Ogino-Knaus and basal temperature method. The first has a high failure rate and has practically been abandoned. The second, more accurate, although presenting notable uncertainties, has the drawback of being extremely impracticable and is often used in combination with the cervical mucous method to increase efficiency;

d) the condom and the diaphragm are too artificial methods of imposing a mechanical barrier, but both of them have important negative psychological effects, causing loss of naturalness and libido. Furthermore, the second requires a great deal of experience on the part of the woman with consequent increase of inhibition on the part of her partner. Lastly, the diaphragm has the snag of having to remain in place for many hours after sexual relation, of requiring the use of a spermicidal cream for greater security and of causing vaginal irritation.

Finally we should mention sterilisation, either permanent or reversible and abortion for medical (therapeutical) reasons and non medical reasons, even though these methods cannot properly be considered as family planning.

The device according to the present invention has the main purpose of overcoming the disadvantages and drawbacks which are typical of the known techniques, to give a good solution for the remote measurement of body temperature with high accuracy and to give a reliable means for family planning based on the measurement of the body temperature of a woman in the basal zone.

This can be achieved by means of a device having the features described in claim 1.

The dependent claims outline advantageous forms of embodiment of the invention.

The device according to the invention is based on the measurement of the electromagnetic energy emitted by each body that has has a temperature above the absolute zero (-273 °C), according to the well known in physics Planck's Law.

In particular, the temperatures of human bodies have the maximum of emission in the medium/far infrared range, more precisely at about 10 $\mu$m, according to Wien's Law.

The device according to the invention includes reference units for temperature and permits us to work in the field of natural methods of birth control as it carries out a remarkable improvement, in accuracy and practicalness, of the method based on the measurement of the basal zone and, in particular, of the variation of the temperature caused by ovulation, said method being normally carried out with the technique of using a thermometer filled with mercury. Moreover, the device according to the invention performs the temperature measurement of the zones under examination in an automatic way, without surface contact, in real time, and permits women the choice of wearing light clothes.

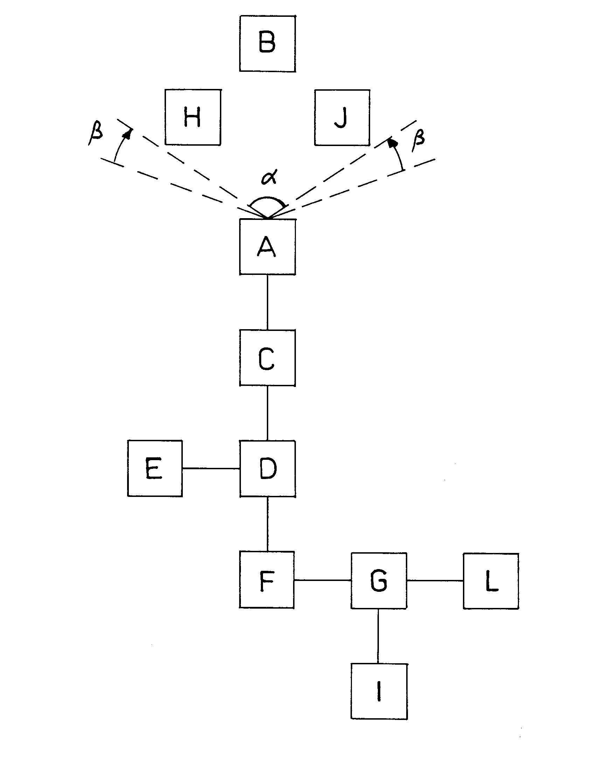

Other features and advantages of the invention will become apparent by reading the following description of a form of embodiment of the invention, given as a non-limiting example, with the help of the figure shown in the attached sheet, which shows, by means of a block diagram the principle of functioning of the device according to the invention.

In order to obtain a measurement as accurate as possible, the device according to the invention and the human body under examination are placed in an air-conditioned room; moreover, the person is in a very relaxed state, as consequent to Schultz's training, so that emotions do not affect the temperature of the body.

In the figure, the device according to the invention comprised an objective (A) suitable for picking-up, filtering and focussing the electromagnetic energy emitted by the basal zone of the person (B) under examination in the angular field: $\alpha$ in the horizontal direction and $\beta$ in the vertical one.

This energy is collected, with the aid of suitable scanning means (C) of the angular field, on a sensor (D) for detecting infrared energy which is suitably cooled by a cryogenic unit (E).

The sensor (D) is connected to an electronic unit (F) that performs the filtering of the signal by means of suitable algorhytms and, in combination with the scanning unit (C) capable of producing an electronic signal which, for easier elaboration, is made in accordance to a standard TV signal and which represents the information concerning the temperatures of the basal zones of the person (B) under examination.

This signal is like a normal black/white TV signal, with grey tones corresponding to differnt levels of temperature. The references in temperature of the signal are two sources of known temperature (H, J).

A suitable electronic unit (G) for the elaboration of the signal generates a TV signal with pseudo-colors corresponding to the levels of temperature in the scanned scene and according to the largest variations of the temperature of the body that are normal in the natural phenomenon under observation and according to the standards in medicine.

The signal is shown in luminous form and can be recorded (I); it is also presented on paper by means of a printing unit (L).

The measurement of temperature is suitable, in accuracy and resolution, for the above-mentioned purposes.

The composition of each unit belonging to the the device according to the invention is described as follows.

The block A is constituted by an optical unit suitable for picking-up, filtering and focussing the energy emitted by the bodies that are in the angular field $\alpha$, $\beta$ and, in particular, by the basal zone of the person under examination.

Lenses are made with germanium and are provided with suitable coatings to increase the transmission of energy.

The electromechanical scanning unit C is consituted, in this specific example, by a rotating drum; in this way the sensor is allowed to explore the predetermined field of view and to prepare the suitable form of information in such a way as to facilitate the generation of a standard TV signal composed of frames and horizontal lines.

The block D is composed of an unit including a photovoltaic infrared sensor which comprises, as an example, SPRITE HgCdTe (mercury cadmium telluride) elements and which has a high sensitivity for wavelengths of about 10 $\mu$m.

This block D is connected to the block E that has the task of cooling the infrared sensor.

The cooling, down to a temperature of about -200$^\circ$C, is operated either with an open loop, like Joule-Thomson, or with a closed loop, like Stirling.

The block F is the electronic unit of the infrared sensor; it provides for the filtering of the received data and for the preparation of the electronic signal according to TV standard.

The block G is constituted by a unit for the elaboration of the electronic image in order to facilitate interpretation.

As a simplified example, the block G provides for the elaboration of the received image according to a scale of colours suitable for the resolution in temperature and for the standards of medicine.

The calibration of the scale of temperature is done periodically, for example checking the two units H and J, that are the sources of reference in temperature, in the field of view of the device and which are constituted, for instance, by thermostatized black bodies.

The block I constitutes the unit for the presentation of the image in luminous form, for instance on a TV monitor, in order to have an immediate presentation and for the recording of said image in a suitable form, e.g. on a magnetic tape.

Finally, the block L comprises a unit that permits the presentation of the data on paper in order to make the evaluation easier also in comparison with the results obtained in other moments by the person under examination.

The device according to the invention has been described with reference to an advantageous form of embodiment.

However, it is clear that said device has many other applications such as, for example, measuring of localized body temperature in breast cancer prevention, headache and neuropathies diagnostic methods, etc.

## Claims

1. Device for the remote measurement of the temperature of the body, advantageously device for the remote measurement of the temperature of the basal zone of women (B), characterised in that it comprises first means (A) for filtering and focussing the electro-magnetic energy emitted by a zone of the body included inside of a predetermined solid angular field $(\alpha, \beta)$ in the person (B) under examination, second means (C) for scanning said angular field $(\alpha, \beta)$, third means (D) for detecting said electro-magnetic energy connected to said second means (C), fourth means (F) of elaboration of data connected to said third means (D) and suitable for filtering the signal coming from said third means (D) and moreover suitable for generating an electronic signal representing the temperatures of the zone under examination of said person, finally comprising fifth means (G,I,L) suitable for giving in a visual form and/or to record and/or to print on paper the information supplied by said electronic signal.

2. Device according to claim 1, characterised in that said third detecting means (D) are connected to sixth means (E) suitable for cooling said third means (D).

3. Device according to anyone of the preceding claims, characterised in that said first means (A) are constituted by an optical unit suitable for filtering and focussing the electro-magnetic energy emitted by the bodies located within said angular field $(\alpha, \beta)$, the lenses of said optical unit being advantageously made in germanium and provided with suitable coatings for increasing the transmission of energy.

4. Device according to anyone of the preceding claims, characterised in that said second scanning means (C) are constituted by an optical-mechanical unit including a rotating drum.

5. Device according to anyone of the preceding claims, characterised in that said third means (D) are constituted by a unit including at least one infrared sensor, advantageously a photovoltaic sensor.

6. Device according to claim 5, characterised in that said sensor includes mercury cadmium telluride SPRITE elements whose sensitivity is higher at wavelenghts of about 10 $\mu$m.

7. Device according to anyone of the preceding claims, characterised in that said fourth means (F) of elaboration of data comprises an electronic unit for the filtering of data coming from said third means (D) and for generating an electronic signal according to TV standard.

8. Device according to anyone of the preceding claims, characterised in that said fifth means (G,I,L) includes a unit (G) for the elaboration of the electronic signal, a unit (I) for the display and/or the recording and/or a unit (L) for the presentation on paper of the information supplied by said electronic signal.

9. Device according to anyone of claims 2 to 8, characterised in that said sixth means (E) includes a cooling unit operating at a temperature of about -200 $^\circ$C.

10. Device according to claim 9, characterised in that said cooling unit includes either an open loop cycle, like Joule-Thomson, or a closed loop cycle, like Stirling.

11. Device according to anyone of the preceding claims, characterised in that it comprises a pair of elements (H,J), advantageously a pair of thermostatized black bodies, giving the reference temperatures for carrying out the temperature measurement.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | SPIE 15TH ANNUAL TECHNICAL SYMPOSIUM PROCEEDINGS vol. 3, 17 September 1970, ANAHEIM, CA (US) pages 191 - 205; J.A. WIELAND: 'An Evaluation of Current Status of Medical Thermography' * page 194 - page 202; figures 7A,13 * | 1-3,5-10 | A61B5/00 A61B10/00 |
| X | US-A-3 862 423 (A.F. KUTAS ET AL.) * column 3, line 23 - column 4, line 8 * * column 11, line 25 - column 12, line 3 * * figures 1-3 * | 1,2,5-7 | |
| A | BRITISH JOURNAL OF PHOTOGRAPHY. vol. 116, no. 5670, 21 March 1969, LONDON GB pages 276 - 278; S.W. BOWLER: 'Thermography' | 1,2,4,5 ᴓ | |
| A | * page 276 - page 278; figure 4 * | 8-10 | |
| A | IEEE TRANSACTIONS ON BIO-MEDICAL ENGINEERING. vol. BME27, no. 5, May 1980, NEW YORK US pages 242 - 248; D.C. JEUTTER ET AL.: 'A Modular Expandable Implantable Temperature Biotelemeter' * page 246 - page 247 * | 1 | TECHNICAL FIELDS SEARCHED (Int. Cl.5 ) A61B |

The present search report has been drawn up for all claims

| Place of search | Date of completion of the search | Examiner |
|---|---|---|
| THE HAGUE | 15 NOVEMBER 1991 | RIEB K.D. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document